(19) **Europäisches Patentamt / European Patent Office / Office européen des brevets**

(11) **EP 2 554 425 A1**

(12) # EUROPEAN PATENT APPLICATION

(43) Date of publication:
**06.02.2013 Bulletin 2013/06**

(51) Int Cl.:
**B60L 11/18** (2006.01)          **G01N 33/00** (2006.01)
**H01M 8/04** (2006.01)

(21) Application number: **12178146.2**

(22) Date of filing: **27.07.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.08.2011 JP 2011169533**

(71) Applicants:
• **Hitachi Automotive Systems, Ltd.**
  **Hitachinaka-shi**
  **Ibaraki**
  **312-8503 (JP)**
• **Honda Motor Co., Ltd.**
  **Tokyo 107-8556 (JP)**

(72) Inventors:
• **Kanno, Shuichi**
  **Tokyo, 100-8220 (JP)**

• **Yoshii, Yasuo**
  **Tokyo, 100-8220 (JP)**
• **Onozuka, Junji**
  **Ibaraki, 312-8503 (JP)**
• **Soshino, Masahiko**
  **Ibaraki, 312-8503 (JP)**
• **Oishi, Hidetoshi**
  **Saitama, 351-0113 (JP)**
• **Okajima, Kazuhiro**
  **Saitama, 351-0113 (JP)**
• **Tsukabayashi, Shunji**
  **Saitama, 351-0113 (JP)**

(74) Representative: **MERH-IP**
  **Matias Erny Reichl Hoffmann**
  **Paul-Heyse-Strasse 29**
  **80336 München (DE)**

(54) **Hydrogen sensing device**

(57)    The invention provides a hydrogen sensing device 100, 100A capable of preventing sensor sensitivity decreases.

A hydrogen sensor 150 detects the hydrogen in an exhaust pipe EP and is installed in a divergent pipe DP. Flowing through the exhaust pipe is off-gas discharged from the fuel cell of a fuel cell vehicle. In order to decompose the siloxanes contained in the gas, a siloxane decomposing material 130 is placed upstream of the hydrogen sensor 150 inside the divergent pipe DP. Also, an $SiO_2$ capturing material 140 is placed between the siloxane decomposing material 130 and the hydrogen sensor 150, so that the $SiO_2$ capturing material can capture the $SiO_2$ resulting from the decomposition of siloxanes by the siloxane decomposing material 130.

## FIG. 2

**Description**

BACKGROUND OF THE INVENTION

1. Field of the Invention

The present invention relates to hydrogen sensing devices for detecting hydrogen and particularly to a hydrogen sensing device suitable for detecting the hydrogen in off-gas discharged from a fuel cell vehicle.

2. Description of the Related Art

The application of a hydrogen sensing device to a fuel cell vehicle is now being considered in order to detecting the hydrogen in off-gas. It is now known that impurities such as organosilicon compounds (e.g., siloxanes) lead to the poisoning of the catalyst, affecting hydrogen-detecting performance.

To counter this, the method disclosed in JP-2008-101207-A is designed to capture the organosilicon complexes contained in a gasoline distillate. Under this method, when organosilicon compounds are contained in a gasoline distillate as impurities, an alumina material supporting alkali-based metal is used to remove those impurities.

SUMMARY OF THE INVENTION

Siloxanes are now known to be generated from Si-based sealing materials or packing materials, depending on use conditions. Because such materials are used at various locations of a vehicle due to their superior characteristics, difficulty is involved in removing the sources of siloxanes.

An object of the present invention is thus to provide a hydrogen sensing device capable of preventing sensor sensitivity decreases.

To achieve the above object, the present invention is a hydrogen sensing device to be installed on a fuel cell vehicle, the device comprising: a hydrogen sensor for detecting hydrogen; and / or a siloxane decomposing material, installed upstream of the hydrogen sensor, for decomposing siloxanes in gas flowing through a fuel cell exhaust pipe of the fuel cell vehicle.

With the above configuration, sensor sensitivity can be prevented from decreasing.

The present invention provides a hydrogen sensing device capable of preventing sensor sensitivity decreases.

BRIEF DESCRIPTION OF THE DRAWINGS

FIG. 1 is a block diagram illustrating the configuration of a fuel cell vehicle on which a hydrogen sensing device according to an embodiment of the invention is installed;
FIG. 2 is a block diagram illustrating the configuration of the hydrogen sensing device;
FIG. 3 is a cross-sectional view illustrating the detailed configuration of the hydrogen sensing device;
FIG. 4 is a graph illustrating the effects of a siloxane decomposing material used for the hydrogen sensing device; and
FIG. 5 is a block diagram illustrating the configuration of a hydrogen sensing device according to another embodiment of the invention.

DESCRIPTION OF THE PREFERRED EMBODIMENTS

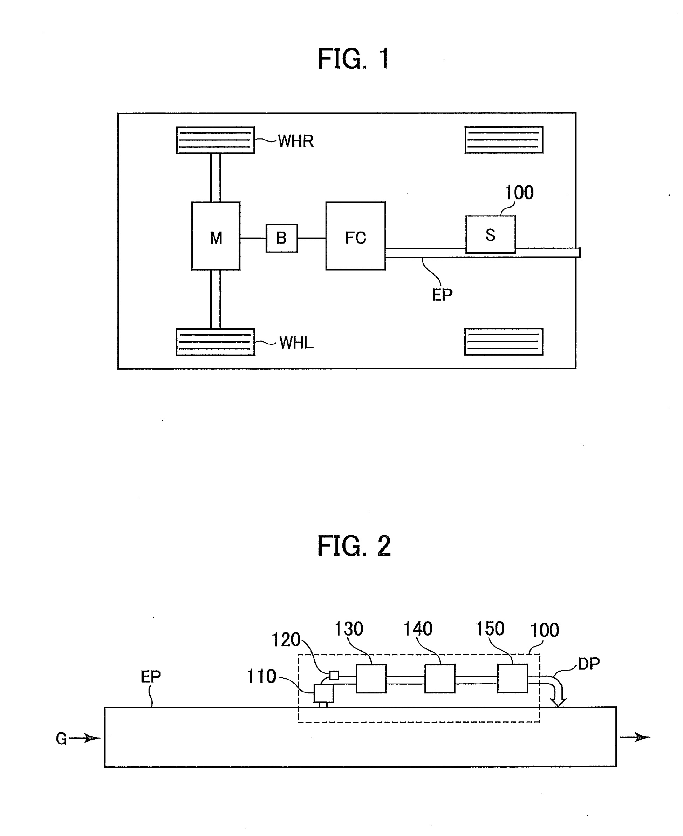

With reference to FIGS. 1 through 4, a hydrogen sensing device according to an embodiment of the invention will be described.

First described with reference to FIG. 1 is the configuration of a fuel cell vehicle that has the hydrogen sensing device installed.

FIG. 1 is a block diagram illustrating the configuration of the fuel cell vehicle.

A fuel cell FC generates power using hydrogen and oxygen as fuels. The off-gas from the fuel cell FC is discharged through an exhaust pipe EP to the outside. The power generated by the fuel cell FC is accumulated by a battery B. The accumulated power is used to rotate a motor M, thereby also rotating wheels WHR and WHL to move the vehicle forward or backward.

To detect the concentration of hydrogen in the off-gas, a hydrogen sensing device 100 is installed on the exhaust pipe EP.

It is to be noted that the off-gas discharged from the fuel cell FC is discharged from the oxygen electrode of that cell, which electrochemically generates power by the fuel electrode receiving hydrogen and the oxygen electrode receiving oxygen as reactive gases.

Next described with reference to FIG. 2 is the configuration of the hydrogen sensing device 100 of the invention.

FIG. 2 is a block diagram illustrating the hydrogen sensing device 100.

The hydrogen sensing device 100 is attached to the exhaust pipe EP to detect the concentration of hydrogen in off-gas G. This gas G is discharged from the fuel cell and contains hydrogen and siloxanes.

A divergent pipe DP is also attached to the exhaust pipe EP. Part of the gas G that flows through the exhaust pipe EP is directed into this divergent pipe DP.

From the upstream gas direction to the downstream gas direction, the hydrogen sensing device 100 includes the following components: a heater 110; a decomposition aid inlet 120; a siloxane decomposing material 130; an $SiO_2$ (silicon dioxide) capturing material 140; and a hydrogen sensor 150.

The hydrogen sensor 150 detects hydrogen when it comes into contact with the sensor 150. The sensor 150 can be a combustion-type hydrogen sensor, which is designed to combust hydrogen and detect its volume based on the calorific value obtained during the combustion. It is also possible to use a semiconductor-based hydrogen sensor in place of the combustion-type hydrogen sensor.

During the detection of the hydrogen in the exhaust gas G, the sensitivity of the hydrogen sensor 150 may decrease when siloxanes come into contact with the sensor 150. Thus, proper operation of the sensor 150 requires prevention of sensor sensitivity decreases. This means that the siloxanes (i.e., impurities) in the gas G need to be reduced in amount before they touch the sensor 150.

As a result of our intensive study, we have found that the sensor sensitivity can be prevented from decreasing when the impurities in the gas G, which also contains hydrogen to be detected by the hydrogen sensor 150, are caused to touch the siloxane decomposing material 130 under proper conditions before they flow into the hydrogen sensor 150. By doing so, the impurities can be removed from the gas G, leading to prevention of sensor sensitivity decreases.

Siloxanes (i.e., impurities) are organosilicon compounds that have the basic structure of Si and O and refer to any compound having the Si-O-Si bonding. Low-molecular siloxanes, in particular, may often lead to catalyst poisoning. Low-molecular siloxanes are chemical compounds composed of successively bonded structural units of $-OSi(CH_3)_2-$. The number of these structural units determines the size of a siloxane compound; for example, siloxane D5 is a chain compound composed of five of those units. A compound with one or two of those units is unable to form a chain structure and thus forms a linear structure. The ends of such a compound are either $-CH_3$ or $-OH$.

The gas G containing siloxanes and hydrogen is caused to flow into the siloxane decomposing material 130. After passing through the siloxane decomposing material 130, the gas G collides with the hydrogen sensor 150, where the hydrogen is measured. Note that before the gas G is introduced into the siloxane decomposing material 130, a decomposition aid can be mixed into the gas G through the decomposition aid inlet 120. It is also preferred to install the $SiO_2$ capturing material 140 between the siloxane decomposing material 130 and the hydrogen sensor 150 in the event that decomposition of the siloxanes produces $SiO_2$ and that this $SiO_2$ affects the performance of the hydrogen sensor 150. The $SiO_2$ capturing material 140 serves the function of capturing siloxanes that have not been decomposed by the siloxane decomposing material 130.

The siloxane decomposing material 130 could be any as long as it is capable of decomposing siloxanes; for example, it may be composed of a metal oxide that aids hydrolysis or oxidative decomposition of siloxanes. Hydrolysis is preferred to be aided if the siloxanes-containing gas G includes $H_2O$, and oxidative decomposition is preferred to be aided if the gas G includes $O_2$. Hydrolysis using $H_2O$ is more desirable because it increases byproduct selectivity.

The siloxane decomposing material 130 needs to have acid sites as its chemical characteristics. Also, the quantity of acid sites per unit surface area is preferred to be large. Typically, the $NH_3$ adsorption method is used to measure the quantity of acid sites in a material. It is preferred that the siloxane decomposing material 130 have an acid site quantity of 0.0042 $\mu mol/m^2$ or greater, which can be calculated from two values: the acid site quantity obtained from the $NH_3$ adsorption method; and the specific surface area of the material 130 measured under the BET method.

The following shows how we measured the quantity of acid sites. For the measurement, we used Metal Dispersion Analyzer BELCAT-A (Bell Japan, inc.). Particles with diameters of 0.5 to 1 mm were used as the siloxane decomposing material 130. The amount used as a sample weighted 0.05 g. The measurement consisted of 1) pretreatment, 2) $NH_3$ adsorption, and 3) temperature raising & $NH_3$ desorption. In the pretreatment, 50 ml of He was delivered to the analyzer per minute as the process gas, and the inner temperature of the analyzer was raised 10°C per minute, starting from room temperature to 500°C, which was maintained for 60 minutes. Thereafter, the inner temperature of the analyzer was lowered to 100°C by natural cooling. In the $NH_3$ adsorption, 50 ml of He having a 5-vol/% $NH_3$ concentration was delivered per minute at 100°C, and the delivery was continued for 30 minutes. After the adsorption of $NH_3$, the gas was switched to He, and 50 ml of He was delivered per minute, which was continued for 15 minutes. In the temperature raising and $NH_3$ desorption, 30 ml of He was delivered per minute, and the inner temperature of the analyzer was raised 10°C per minute, starting from 100°C to 700°C. After the temperature was raised to 700°C, it was maintained until the amount of $NH_3$ was lowered to the amount measured before the $NH_3$ adsorption. The $NH_3$ desorbed

during the temperature raising was measured with the use of a gas chromatographic analyzer, and during the chromatographic analysis, 30 ml of He was delivered per minute as the carrier gas. The measurement gas was directly introduced into a TCD detector without using a filling column, and the temperature of the TCD detector was set to 100°C. The amount of adsorbed $NH_3$ was the total amount of $NH_3$ detected during the temperature raising and the $NH_3$ desorption.

The siloxane decomposing material 130 is also preferred to have a large specific surface area. The specific surface area of the siloxane decomposing material 130 becomes large when the material 130 has a microporous or mesoporous structure. The siloxane decomposing material 130 can also be supported onto a material with a large specific surface area. For the purpose of achieving a large specific surface area, it is also possible to bond the decomposing material 130 to a material with a large specific surface area and then oxidize the resultant substance to form a composite oxide.

In addition, by controlling the hydrophobicity or hydrophilicity of the surface of the siloxane decomposing material 130, siloxane capturing capabilities can be improved. For instance, hydrophilic components may be supported onto the material surface to control the surface properties. It is also possible for the siloxane decomposing material 130 to support hydrophobic components such as organic groups and the like. Because siloxanes have hydrophilic Si groups and hydrophobic organic groups, an increase in the hydrophilicity of the material 130 enhances the responsiveness of the material 130 to Si groups while an increase in the hydrophobicity of the material 130 enhances the capability of the material 130 to capture organic groups. It is also possible for the siloxane decomposing material 130 to support both of a hydrophobic substance and a basic substance.

The substances that meet the above-mentioned conditions include $TiO_2$, $ZrO_2$, NiO, ZnO, CoO, $Fe_2O_3$, and the like and can thus be used as the siloxane decomposing material 130. These substances can sometimes meet the aforementioned preferred acid site quantity by themselves, but when it is not met, hydroxyl groups can be bonded by surface treatment, thereby increasing the quantity of acid sites. Those substances can also be used as composite oxides, not as a single substance.

The siloxane decomposing material 130 can be shaped into grains, a column, pellets, or the like. It is also possible to coat the surfaces of a ceramic honeycomb-shaped structure or the surfaces of metal wires with the siloxane decomposing material 130.

It is preferred to put the siloxane decomposing material 130 into a porous cartridge, so that siloxane decomposing material 130 can be replaced with ease.

A decomposition aid to be added from the decomposition aid inlet 120 need not be present in the gas G from the beginning, which contains hydrogen to be detected by the hydrogen sensor 150 and siloxanes. It is preferred to add a decomposition aid to the gas G before the siloxanes in the gas G reaches the siloxane decomposing material 130. To aid hydrolysis, $H_2O$ can be added to the gas G as a decomposition aid. To aid oxidative decomposition, $O_2$ can be added to the gas G. As above, selection of a decomposition aid depends on the decomposing material 130 used and associated decomposition reactions.

It is preferred that Si in siloxanes be converted into $SiO_2$. Adjacent $SiO_2$ molecules are silicate-bonded to one another at a temperature of 700°C or thereabout, forming an amorphous film. But at a low temperature, $SiO_2$ has less poisoning influence on the siloxane decomposing material 130 than siloxanes.

The hydrogen sensing device 100 is operated under optimal conditions although reaction temperatures may vary depending on the decomposing material 130 used. To increase siloxane decomposition efficiency, the temperature of the siloxane decomposing material 130 needs to be raised. At a temperature of 300°C or thereabout, a decomposition rate of almost 100% can be achieved. To increase the temperature of the decomposing material 130, the temperature of the gas G following into the decomposing material 130 can be raised. Alternatively, the temperature of the decomposing material 130 itself can be raised. When the temperature of the gas G is to be increased, the heater 110 (heating element) in the divergent pipe DP can be used. When the heater 110 is to be installed inside the divergent pipe DP, the material of its heating element has to be selected carefully so that the heater 110 will not affect the hydrogen to be detected by the hydrogen sensor 150. If the heater 110 causes chemical reactions of the hydrogen, the sensitivity of the hydrogen sensor 150 can be set so as to offset the hydrogen decrease caused by those reactions. If, on the other hand, the temperature of the decomposing material 130 is to be raised, an external heater can be used. It is also possible to heat the decomposing material 130 by covering a heating element with the decomposing material 130 or by embedding a heating element in the decomposing material 130. In those cases, it is preferred to put a heating element into a casing pipe so that the heating element will not be in direct contact with the decomposing material 130.

The $SiO_2$ capturing material 140 is preferred to have a large specific surface area. Examples of the $SiO_2$ capturing material 140 include mesoporous silica, activated carbon, and the like. To increase $SiO_2$ capturing capabilities, the surfaces of such materials can be modified with functional groups having a particular affinity with Si. Substances having affinity with Si include Si and Al. It is also possible to use substances that bonds to $SiO_2$ to form compounds.

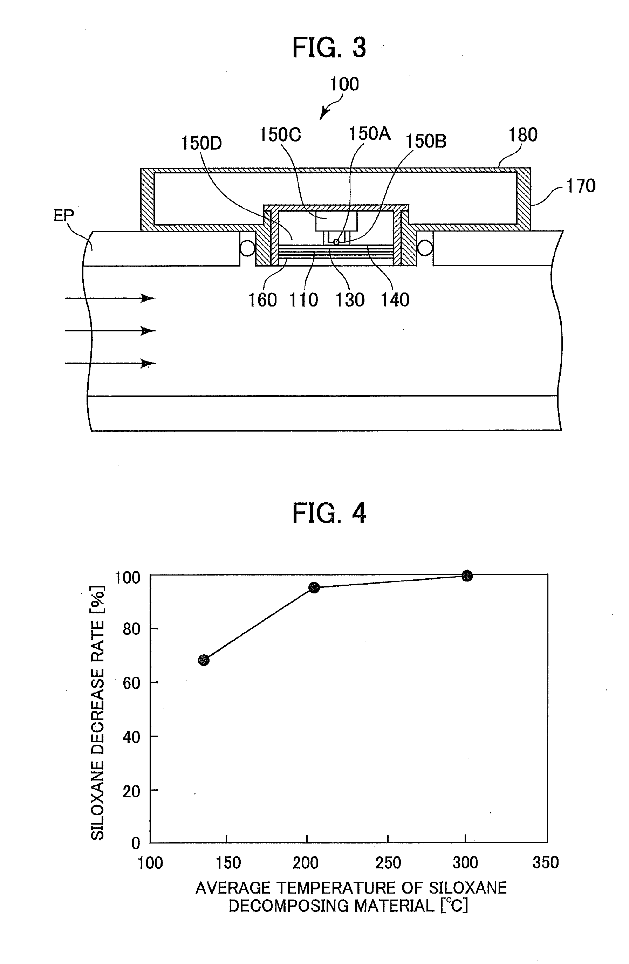

Next described with reference to FIG. 3 is the detailed configuration of the hydrogen sensing device 100 of the invention.

FIG. 3 is a cross-sectional view of the hydrogen sensing device 100.

The hydrogen sensing device 100 is attached to the exhaust pipe EP. The hydrogen sensing device 100 includes a casing 170. The casing 170 houses part of a hydrogen gas detecting chamber 150D. The hydrogen gas detecting chamber 150D has a bottom; thus, the chamber 150D has a substantially U-shaped cross-section. The open end of the chamber 150D communicates with the inner side of the exhaust pipe EP. A hydrogen gas detecting element holder 150C is attached to the bottom of the hydrogen gas detecting chamber 150D, and a hydrogen gas detecting element 150A is attached to the holder 150C via a stem 150B. Note that the hydrogen gas detecting element 150A, the stem 150B, the hydrogen gas detecting element holder 150C, and the hydrogen gas detecting chamber 150D constitute the hydrogen sensor 150 of FIG. 2.

In addition, the following components are installed between the hydrogen gas detecting element 150A and the exhaust pipe EP, starting from the side of the exhaust pipe EP: a water-repellent film 160, the heater 110, the siloxane decomposing material 130, and the $SiO_2$ capturing material 140.

The water-repellent film 160 prevents the moisture in the exhaust pipe EP from reaching the hydrogen gas detecting element 150A. The water-repellent film 160 repels water but lets gas components through. The heater 110, the siloxane decomposing material 130, and the $SiO_2$ capturing material 140 are shaped into plate-like structures and attached to the casing that forms the gas detecting chamber 150D. The siloxane decomposing material 130 is put in a cartridge. Thus, to replace the siloxane decomposing material 130, the cartridge has only to be replaced with a new one. It is preferred that a particular amount of gas come into contact with the hydrogen gas detecting element 150A. To achieve this, the hydrogen gas detecting chamber 150D, the hydrogen gas detecting element holder 150C, or the like may be provided with a gas flow adjusting vent.

Referring now to FIG. 4, the effects of the siloxane decomposing material 130 of the hydrogen sensing device 100 will be described.

FIG. 4 is a graph illustrating the effects of the siloxane decomposing material 130.

The graph was obtained by introducing a reactive gas containing siloxanes into a reaction pipe having the siloxane decomposing material 130 therein and then measuring the concentrations of the siloxanes near the exit of the reaction pipe. The decomposing material 130 used had an acid site quantity of 0.0062 $\mu$mol/m$^2$. The same reactive gas was also introduced into an empty reaction pipe without the siloxane decomposing material 130, and siloxane concentrations were measured near the exit of the empty pipe.

To prepare the reactive gas, 1.88 ml of purified water was first vaporized per minute with the use of a steam generator, and 7.4 ml of dry air was then added to the vapor per minute. Also, 75 ml of hydrogen ($H_2$) and 2576.8 ml of dry air were mixed into the resultant gas per minute. Part of the dry air is used to supply siloxane D5.

The thus-obtained reactive gas was introduced into the siloxane decomposing material 130 when the temperature of the material 130 was 134°C, 203°C, and 300°C. The volume of the siloxane decomposing material used was 2.7 ml (3.59 g).

The reaction pipe with the siloxane decomposing material 130 had a perforated plate at its center, and a 27 mm layer of alumina wool was placed on the perforated plate. The siloxane decomposing material 130 (2.7 ml) was placed on that alumina wool. The layer height of the decomposing material 130 was 15 mm, and the particle diameters of the decomposing material 130 were set to 0.5 to 1.0 mm. The reactive gas introduced into this reaction pipe flows through the siloxane decomposing material 130 and reaches the exit of the pipe.

Siloxane D5, a kind of siloxanes, is in the form of liquid. This liquid was heated to a particular temperature, and dry air was supplied to the heated liquid in the form of bubbles. The resultant siloxane D5 was supplied to the reactive gas using the vapor pressure of the siloxane D5.

The relationship between the siloxane decomposing material 130 and the volume of the reactive gas is represented by the space velocity of Equation 1 and the linear velocity of Equation 2.

$$\text{Space velocity (h}^{-1}\text{)} = \text{reactive gas volume (ml/h}^{-1}\text{)} /$$
$$\text{decomposing material volume (ml)} \quad \text{[Equation 1]}$$

$$\text{Liner velocity (m/s)} = \text{reactive gas flow rate (m}^3\text{/s)} /$$
$$\text{decomposing material cross-sectional area (m}^2\text{)} \quad \text{[Equation 2]}$$

The relationship between the siloxane decomposing material 130 and the volume of the reactive gas was such that the space velocity of Equation 1 was 111.084h$^{-1}$ and the linear velocity of Equation 2 was 47.2 m/s.

Table 1 below shows the results of the siloxane measurement.

[Table 1]

| D type | Average temperature of decomposing material = 134°C | | Average temperature of decomposing material = 203°C | | Average temperature of decomposing material = 300°C | |
|---|---|---|---|---|---|---|
| | Entrance (volppb) | Exit (volppb) | Entrance (volppb) | Exit (volppb) | Entrance (volppb) | Exit (volppb) |
| Hexamethyldisiloxane | 6.3 | 5.7 | 4.7 | 4.0 | 0.0 | 0.0 |
| D3 | 3.0 | 2.0 | 3.4 | 1.1 | 6.1 | 1.0 |
| D4 | 3.1 | 2.1 | 4.2 | 2.0 | 5.9 | 1.5 |
| D5 | 1553.0 | 483.0 | 1464.0 | 60.0 | 1286.0 | 6.7 |
| D6 | 0.5 | 0.6 | 0.8 | 0.6 | 0.5 | 0.4 |
| D7 | 0.1 | 0.1 | 0.1 | 0.1 | 0.1 | 0.0 |
| D8 | 0.1 | 0.1 | 0.0 | 0.0 | 0.0 | 0.0 |
| Total | 1566.1 | 493.5 | 1477.2 | 67.8 | 1298.6 | 9.6 |

"Total" represents the sum of siloxane concentrations. As can be seen, there were drastic decreases in the respective totals of siloxane concentrations measured at the exits of the 134°C, 203°C, and 300°C decomposing materials 130.

The graph of FIG. 4 illustrates the dependency of siloxane decrease rates on temperature, which rates were calculated from the concentrations of Table 1 and gas flow rates. When the average temperature of the decomposing material 130 was 134°C, 203°C, and 300°C, the siloxane decrease rate was 68.5%, 95.4%, and 99.3%, respectively. In addition to the siloxane measurement, we also conducted an analysis of methane ($CH_4$), and $CH_4$ was found in a gas whose siloxane concentrations were reduced, which suggests the decomposition of siloxane D5.

As described above, the present embodiment of the invention prevents the sensitivity of the hydrogen sensor from decreasing even when a gas containing hydrogen to be detected by the hydrogen sensor includes organosilicon compounds such as siloxanes.

Moreover, due to the installation of the decomposition aid inlet 120 and the siloxane decomposing material 130 on the divergent pipe DP, a smaller amount of gas is required for the hydrogen sensor 150. Accordingly, the amounts of decomposition aids and the siloxane decomposing material 130 can also be reduced.

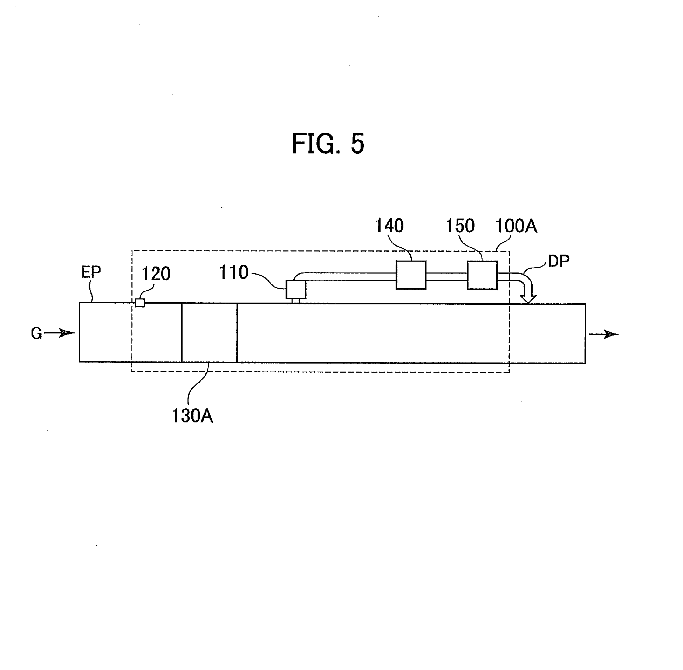

Next described with reference to FIG. 5 is the configuration of a hydrogen sensing device according to another embodiment of the invention.

FIG. 5 is a block diagram illustrating that hydrogen sensing device. Note that the same reference numerals as those used in FIG. 2 represent identical components.

The hydrogen sensing device 150A of this embodiment has the basically same structure as that of the hydrogen sensing device of FIG. 2. The differences between FIG. 5 and FIG. 2 are that, in FIG 5, a siloxane decomposing material 130A is installed inside the exhaust pipe EP through which the gas G containing siloxanes and hydrogen flows, and the decomposition aid inlet 120 is installed upstream of the siloxane decomposing material 130A.

The gas G containing hydrogen to be detected by the hydrogen sensor 150 is directed into the exhaust pipe EP and flows through the siloxane decomposing material 130A installed inside the exhaust pipe EP. Part of the gas G that has flowed past the decomposing material 130A is directed to the divergent pipe DP which has the $SiO_2$ capturing material 140 and the hydrogen sensor 150 installed. The gas G that has flowed past the hydrogen sensor 150 is directed back to the exhaust pipe EP, but it can also be discharged through a different pipe. The installation of the $SiO_2$ capturing material 140 is optional.

As above, this embodiment of the invention also prevents the sensitivity of the hydrogen sensor from decreasing even when a gas containing hydrogen to be detected by the hydrogen sensor includes organosilicon compounds such as siloxanes.

Features, components and specific details of the structures of the above-described embodiments may be exchanged or combined to form further embodiments optimized for the respective application. As far as those modifications are apparent for an expert skilled in the art they shall be disclosed implicitly by the above description without specifying explicitly every possible combination.

**Claims**

1. A hydrogen sensing device to be installed on a fuel cell vehicle, the device comprising:

   a hydrogen sensor (150) for detecting hydrogen; and
   a siloxane decomposing material (130, 130A), installed upstream of the hydrogen sensor (150), for decomposing siloxanes in gas flowing through a fuel cell exhaust pipe (EP) of the fuel cell vehicle.

2. The hydrogen sensing device of claim 1, wherein the hydrogen sensor (150) detects hydrogen in off-gas flowing through the fuel cell exhaust pipe (EP).

3. The hydrogen sensing device of claim 2, further comprising:

   a divergent pipe (DP) for receiving part of the off-gas flowing through the fuel cell exhaust pipe (EP),
   wherein the hydrogen sensor (150) is installed in the divergent pipe (DP).

4. The hydrogen sensing device of claim 3, wherein the siloxane decomposing material (130, 130A) is installed in the divergent pipe (DP).

5. The hydrogen sensing device of at least one of claims 1 to 4, further comprising:

   a silicon dioxide capturing material (140), placed between the siloxane decomposing material (130, 130A) and the hydrogen sensor (150), for capturing silicon dioxide resulting from decomposition of siloxanes by the siloxane decomposing material (130, 130A).

6. The hydrogen sensing device of at least one of claims 1 to 5, further comprising:

   an inlet (120), installed upstream of the siloxane decomposing material (130, 130A), for injecting a decomposition aid therethrough to aid the decomposition of siloxanes by the siloxane decomposing material (130, 130A).

7. The hydrogen sensing device of at least one of claims 1 to 6, further comprising:

   a heater (110), installed upstream of the hydrogen sensor (150), for heating off-gas heading toward the hydrogen sensor (150).

8. The hydrogen sensing device of at least one of claims 1 to 3, wherein the siloxane decomposing material (130, 130A) is installed in the fuel cell exhaust pipe (EP).

9. The hydrogen sensing device of at least one of claims 1 to 8, wherein the hydrogen sensor (150) detects hydrogen based on a calorific value obtained from combustion reactions.

10. The hydrogen sensing device of claim 1, further comprising:

    a casing (170) having a hydrogen gas detecting chamber (150D),
    wherein the hydrogen sensor (150) is secured at the bottom of the hydrogen gas detecting chamber (150D), and the siloxane decomposing material (130, 130A) is installed on the open end side of the hydrogen gas detecting chamber (150D).

# FIG. 1

# FIG. 2

# FIG. 3

# FIG. 4

# FIG. 5

Europäisches
Patentamt

European
Patent Office

Office européen
des brevets

## EUROPEAN SEARCH REPORT

**Application Number**

EP 12 17 8146

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 60 222144 A (MATSUSHITA ELECTRIC WORKS LTD) 6 November 1985 (1985-11-06) * the whole document * | 1-10 | INV. B60L11/18 G01N33/00 |
| X | JP 2002 052338 A (NEW COSMOS ELECTRIC CO) 19 February 2002 (2002-02-19) * figure 7 * | 1-10 | ADD. H01M8/04 |
| X | JP 8 247982 A (NEW COSMOS ELECTRIC CO) 27 September 1996 (1996-09-27) * paragraph [0012] * * pages --; figures * | 1-10 | |
| A | US 2008/092738 A1 (NEDEZ CHRISTOPHE [FR]) 24 April 2008 (2008-04-24) * paragraph [0006] * | 1-10 | |
| A | US 2009/035184 A1 (KODA HIROSHI [JP] ET AL) 5 February 2009 (2009-02-05) * paragraph [0016] - paragraph [0021] * * figures 3-5 * | 1-10 | TECHNICAL FIELDS SEARCHED (IPC) G01N |

The present search report has been drawn up for all claims

| Place of search | Date of completion of the search | Examiner |
|---|---|---|
| The Hague | 29 November 2012 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 17 8146

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2012

| Patent document cited in search report | | Publication date | Patent family member(s) | | Publication date |
|---|---|---|---|---|---|
| JP 60222144 | A | 06-11-1985 | NONE | | |
| JP 2002052338 | A | 19-02-2002 | JP | 4542248 B2 | 08-09-2010 |
| | | | JP | 2002052338 A | 19-02-2002 |
| JP 8247982 | A | 27-09-1996 | JP | 3197455 B2 | 13-08-2001 |
| | | | JP | 8247982 A | 27-09-1996 |
| US 2008092738 | A1 | 24-04-2008 | BR | PI0704030 A | 03-06-2008 |
| | | | CA | 2606889 A1 | 18-04-2008 |
| | | | CN | 101186839 A | 28-05-2008 |
| | | | EP | 1918005 A1 | 07-05-2008 |
| | | | FR | 2907348 A1 | 25-04-2008 |
| | | | JP | 2008101207 A | 01-05-2008 |
| | | | US | 2008092738 A1 | 24-04-2008 |
| US 2009035184 | A1 | 05-02-2009 | US | 2009035184 A1 | 05-02-2009 |
| | | | WO | 2007097025 A1 | 30-08-2007 |
| | | | WO | 2007099933 A1 | 07-09-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2008101207 A